Europäisches Patentamt

**European Patent Office**

Office européen des brevets

(19)

(11) Publication number : **0 434 749 B1**

(12) # EUROPEAN PATENT SPECIFICATION

(45) Date of publication of patent specification :
**11.08.93 Bulletin 93/32**

(51) Int. Cl.⁵ : **A61K 37/24**

(21) Application number : **89910909.4**

(22) Date of filing : **14.09.89**

(86) International application number :
**PCT/GB89/01081**

(87) International publication number :
**WO 90/02559 22.03.90 Gazette 90/07**

(54) **PHARMACEUTICAL COMPOSITIONS FOR ELICITING AN IMMUNOSTIMULANT EFFECT.**

(30) Priority : **15.09.88 GB 8821656**

(43) Date of publication of application :
**03.07.91 Bulletin 91/27**

(45) Publication of the grant of the patent :
**11.08.93 Bulletin 93/32**

(84) Designated Contracting States :
**AT BE CH DE FR GB IT LI LU NL SE**

(56) References cited :
**EP-A- 85 220**
**CHEMICAL ABSTRACTS, vol. 78, 1973, Columbus, OH (US); J.D.KELLY et al., p. 321, no. 109187c**
**Martindale, THE EXTRA PHARMACOPOEIA, 28th ed., 1982; pp. 1268-1271, 1275-1276, 1278**

(73) Proprietor : **The Public Health Laboratory Service Board**
**61 Colindale Avenue**
**London NW9 5EQ (GB)**

(72) Inventor : **MELLING, Jack**
**1 Folly Close Old Blandford Road Salisbury Wiltshire (GB)**
Inventor : **STEPHENSON, John, Robert**
**11 Paddock Way Laverstock**
**Salisbury Wiltshire (GB)**

(74) Representative : **Ritter, Stephen David et al**
**Mathys & Squire 10 Fleet Street**
**London EC4Y 1AY (GB)**

EP 0 434 749 B1

Jouve, 18, rue Saint-Denis, 75001 PARIS

## Description

This invention relates to pharmaceutical compositions for eliciting an immunostimulant effect in humans or non-human animals Particularly, but not exclusively the invention relates to vaccine preparations and to pharmaceutical compositions for use in conjunction with vaccine preparations in order to enhance the effectiveness of such preparations.

Many circumstances exist in which it is desirable to produce an immunostimulant effect in an animal. Thus, for example, pathological conditions occur in which an animal's immune response is diminished, as, for example, in the case of chronic infections which HIV (Human Immunodeficiency Virus). Further, it would be desirable to produce an immunostimulant effect when administering antigens in the form of vaccines intended to produce a protective immunological effect. Thus, for example, in order to produce an effective degree of immunisation against many pathological microorganisms (including bacteria, fungae, protozoa, viruses, viroids and prions) it is necessary to inoculate on more than one occasion. Similar considerations apply to so-called "contraceptive vaccines", i.e. vaccines, the use of which produce antibodies against hormones involved in reproduction. The use of such "multi-shot" inoculation regimes is a particular drawback in the veterinary field where the inoculation of large numbers of animals is time-consuming and it is inconvenient to record whether or not a particular animal has received the necessary number of inoculations.

Achieving an enhanced immunological response is a particularly desirable objective, particularly in Third World countries, where "multishot" inoculation procedures are impractical and where effective contraceptive vaccines would be desirable.

Pituitary hormones, including pituitary growth hormone, luteinising hormone, follicle stimulating hormone, thyroid stimulating hormone and prolactin, have widespread use in medicine. For example, pituitary growth hormone is used in the treatment of pituitary dwarfism whereas luteinising hormone, follicle stimulating hormone and prolactin are used in treatment, therapy and diagnosis of conditions (including pregnancy) related to reproduction. Thyroid stimulating hormone is, as its name implies, used in therapy of conditions associated with thyroid deficiency.

Conventionally, such pituitary hormones have been extracted from pituitary tissue obtained from cadavers and from slaughter-house by-products, but recently, pituitary hormones have been produced synthetically using, for example, recombinant DNA techniques and synthetic chemistry techniques. The term "pituitary growth hormone" as used herein includes growth hormones which have been extracted from tissues in which they normally occur as well as synthetic forms, such as those produced by recombinant DNA techniques.

Using recombinant DNA techniques and synthetic chemistry techniques it is possible to produce polypeptides which can either be identical to native polypeptides or differ from native polypeptides by deletions, substitutions and insertions of one or more amino acid residues. In the context of the present invention, the term "pituitary growth hormone" is intended to include not only growth hormones produced by recombinant DNA techniques which are identical to native polypeptides but also such hormones which differ from native polypeptides by up to 30 percent of the native sequence. The term "pituitary growth hormone" as used herein is also intended to include pituitary growth hormones having different patterns of glycosylation from the native hormones, including polypeptides which are totally unglycosylated.

A further class of compounds intended to be covered by the term "pituitary growth hormone" consists of fragments of pituitary growth hormones possessing at least one of the characteristic biological activities of the native hormone. In addition, "pituitary growth hormone" is also intended to include pituitary growth hormones having different patterns of acylation, phosphorylation, sulphation and other forms of post-translational modification. A given polypeptide may be identified as being a pituitary growth hormone in the context of the present invention by reference to the relationship of its structure to a native sequence (as indicated above) and by reference to its possession of at least one of the biological properties of the native hormone in a standard assay.

The present invention finds basis in the surprising discovery that pituitary growth hormones are capable of producing an immunostimulant effect.

Thus according to one aspect of the invention there is provided the use of a pituitary growth hormone in the manufacture of a pharmaceutical composition for eliciting an immunostimulant effect in a human or non-human animal.

Pituitary growth hormone used in accordance with the invention may be characterised in terms of biological activity. Thus, for example, pituitary growth hormone may be defined as a polypeptide capable of stimulating long bone growth and releasing somatomedin. Such activity may be detected by the methods described in Marx et al. (1942), Endocrinology, 30, 1. and Greenspan et al. (1949), Endocrinology, 45, 455. Alternatively, pituitary growth hormone may be defined in terms of its antigenic reaction with anti-sera as described, for example in Rabin (1959), Recent Progress In Hormone Research, 15, 71-105. A further characterisation of pituitary growth hormone is by reference to amino acid sequence. Amino acid sequences of known pituitary growth hormones are given in Li (1975), Hormonal Proteins and Peptides, **3**, 1-40, Academic Press. A description of the biological activities of fragments of pituitary growth hormone is also provided in "Structure and Function in Growth Hormones", Wilhelmi, A.E. Proc of the USP Workshop on Drugs and Reference Standards for Insulin Somatotropin and Thyroid Axis Hormones (1982) Bethesda, Maryland.

According to a preferred aspect of the invention, the immunostimulant effect is produced in a human or non-human animal in which it is desired to raise antibodies against a selected antigen. The selected antigen may, for example, be a component of a vaccine. Examples of typical antigens include proteins, glycoproteins, peptides, glycopeptides and polysaccharides and include such antigens derived from viruses, viroids, prions, bacteria, fungae, protozoa and hormones.

The pituitary growth hormone may be administered prior to, simultaneously with or subsequent to the administration of the antigen. Preferably however the hormone and vaccine comprise a single composition adapted for parenteral administration.

The pharmaceutical preparations according to the invention may comprise a single composition containing both the antigen and the pituitary growth hormone or these two components may be present in the form of a kit comprising separate aliquots thereof.

As indicated above, in carrying out the invention, the pituitary growth hormone is advantageously administered in a form which encourages localised retention. Pharmaceutical conditions embodying such forms (whether they additionally comprise one or more of the aforementioned antigens or not) form a further aspect of the invention.

Thus according to a further aspect of the invention there is provided an injectable pharmaceutical composition for use in conjunction with an antigenic component of a vaccine in order to produce an immunostimulant effect, said pharmaceutical composition comprising an antigen, a pituitary growth hormone and a pharmaceutically acceptable carrier, said carrier (or an optional additional component of the composition) being selected so as to promote localised retention of the pituitary hormone at or adjacent to the injection sites. Preferably, said composition comprises an adjuvant such as, for example, aluminium hydroxide.

In carrying out the method of the invention, it has been found that the desired immunological effect (for example a protective effect in the case of immunisation) is obtainable with only a single injection of antigen.

As indicated below, it is believed that the mode of action of pituitary growth hormones according to the invention is via the cell-mediated immune response (rather than by a humoral or antibody response). A mode of administration of pituitary growth hormones which maximises such a response is preferred, i.e. one in which the pituitary growth hormone is administered in a form which encourages localised retention of the hormone after injection. Such forms include injectable compositions which include known adjuvants such as, for example, aluminium hydroxide.

Pharmaceutical preparations may be produced in accordance with the invention comprising both the pituitary growth hormone and the antigen in a single composition. The antigen may be an antigenic component of a vaccine, for example an antigen capable of producing a protective immunological effect against the causative organisms of herpes, rubella, Japanese encephalitis, Rift Valley Fever, yellow fever, measles, mumps, poliomyelitis, louping I11, dengue fever, lassa fever, Central European Tick - borne encephalitis, Russian Spring-Summer encephalitis, Omsk Haemorrhagic Fever, Kyanasur Forest Disease or Wesselsbron disease. Others include antigens capable of producing a protective immunological effect against such organisms as HIV, influenza virus, parainfluenza virus, Epstein-Barr virus and parvo viruses (e.g. the causative organism of so-called B19 otherwise known as slapped-cheek syndrome or Sixth disease). Further examples include the organisms responsible for foot and mouth disease and canine distemper, respiratory syncitial virus, feline leukemia virus, canine parvo viruses, Neisseria sp., Bordatella Sp., Haemophilus sp. and Mycobacterium sp.

Included amongst the causative organisms referred to specifically above are the so-called "flaviviruses".

Flaviviruses have one, single stranded RNA molecule, approximately 11,000 bases long as a genome. The RNA is positive stranded and has a 5' cap but does not have a 3' polyA tail. This genome codes for 3 virus structural proteins and 7 non-structural proteins and in addition has substantial non-coding regions at both the 5' and 3' ends. The genomic RNA is encapsulated in an icosohedral core of about 30 nanometers in diameter, made up entirely of the C protein. This core is surrounded by the E protein as well as a few molecules of M protein. The virus envelope also contains several molecules of lipid incorporated from the membranes of the infected cell.

The C protein is the only structural component of the core of the virus particle. No other functions are known and it does not appear to stimulate the immune system.

The E protein is glycosylated in most flaviviruses and contains all the biological activities associated with the virus particle, and is a major stimulator of the immune system.

The M protein has no known function at present but is present in low numbers in the virus particle.

The NS1 protein is glycosylated, is a major stimulator of the immune system, and has a limited protective function in some systems, but its function is unknown.

The NS3 and NS5 proteins are probably constituents of the viral replicase, but the functions of NS2a, NS2b, NS4a and NS4b are unknown at present.

The flavivirus group of viruses is responsible for some of the most dangerous and widespread diseases of man and his domestic animals including Yellow fever, Dengue fever, Japanese encephalitis and Louping I11. In spite of good vaccine being available, Yellow fever is still a major public health problem in S.E. Asia, Africa and South America. Dengue fever is of growing concern in S.E. Asia and Central America and has been targeted by WHO as one of the most important viral diseases against which there is no current acceptable vaccine. Although this infection is normally 'flu-like in symptomology, there are increasing incidents of Dengue haemorrhagic fever and Dengue Shock Syndrome being reported; these conditions are frequently life-threatening if not treated immediately. Japanese encephalitis is the most rapidly expanding viral disease in the world at present and again has been targetted by the WHO. Tick-borne encephalitis is a major public health problem in the USSR with mortality rates of up to 40% being reported. This disease is also of growing public concern in most Eastern European countries as well as Austria and West Germany. TBE has been reported in most other European countries although it is only recognised as a veterinary problem (Louping I11) in the United Kingdom. However four or five cases of Louping I11 infection of humans are reported in Scotland every year and these can result in hospitalisation for several months.

The present 17D vaccine against Yellow Fever is one of the safest and most efficacious vaccines developed, but as it is a live, attenuated vaccine it requires a cold chain, which is a problem in tropical third world countries where it is most-needed. Development of a non-living vaccine would almost certainly require a multi-shot regime. The only recognised vaccine against Japanese encephalitis is made from an inactivated, partially purified mouse-brain preparation and is effective, but requires three doses for good protection. A live attenuated vaccine has been developed in China and is widely used there as Japanese Encephalitis is a major public health problem, but this vaccine has yet to be tested in the West. Both the Russian and Austrian vaccines against TBE are also inactivated preparations but are made in avian cells and are purified; these also require 2 to 7 doses for complete protection. The veterinary vaccine against Louping I11 is made in the United Kingdom by Wellcome and is manufactured from infected tissue culture cells, inactivated with formalin and has been subjected to only the most rudimentary purification. This vaccine requires at least three of even more doses for a good protection. At present there is no good vaccine against Dengue fever, although the US army has been working on a vaccine for many years. However a group of attenuated vaccines have been developed in Thailand and these are now under trial by the WHO.

The inactivated vaccines against Japanese encephalitis, TBE and Louping I11 could all benefit from a one-shot inoculation regime and even the live attenuated vaccines against Yellow fever, Dengue and Japanese encephalitis may derive a beneficial effect from co-injection with an immunostimulant.

The areas where single-shot vaccination regimens are almost needed are in the military, veterinary vaccines and rural populations in developing countries; it could also be argued that these groups are the most at risk.

The stimulating effect of human growth hormone (also known as somatotrophin or HGH) in mice is demonstrated in the following Examples.

Example 1 - Demonstration of Immunostimulant Effect

The effect of administering HGH on the effectiveness of a vaccine against Tick-Borne Encephalitis (TBE) vaccine was tested in a murine model. The murine model had been developed by the Molecular Virology Group of PHLS Centre for Applied Microbiology & Research to assess the protective ability of various vaccine preparations (both commercial and experimental) against TBE virus (Neudorfl isolate). From previous experiments it was known that a commercially available TBE virus vaccine required a two dose regimen of at least $5\mu$ g of vaccine in order to give 100 percent protection, whereas a single dose was known to give only 10 to 30 percent protection. In view of this established bank of data it was not necessary to include simultaneous controls in which the efficacy of vaccine alone was tested.

In order to assess the effect of HGH on the efficacy of a single vaccine dose, groups of 15 Balb/C mice were injected intra-peritoneally with various combinations of TBE virus vaccine, HGH and PBS (phosphate buffered saline).

Stock solutions were prepared as follows:

1. PBS - NaCl, 0.139M; KCl, 0.0269M; $Na_2 HPO_4$ ($2H_2O$), 0.0075M, $KH_2PO_4$, 0.00147M.

2. HGH - One vial of Batch Number TPL 29 (four units) was dissolved in 1 ml of sterile glass-distilled water The HGH was a polypeptide obtained from extraction from pituitaris.

3. Vaccine - One vial of "FSME-Immun®" (lot no: 37028601) was diluted 1/20 in PBS.

Combined vaccine/HGH preparations where produced by mixing 6 μl of stock solution (2) with 0.2 ml of stock solution (3).

On 10th February 1988 fifteen Balb/C mice were divided into three groups A, B and C and injected intra-peritoneally (i.p.) as follows:

Group A - 0.2 ml of vaccine + HGH

Group B - 0.2 ml of PBS + HGH

Group C - 0.2 ml of vaccine + HGH.

On 24th February 1988 three mice from each of groups A, B and C were bled and the remainder of group A were vaccinated with vaccine and HGH as on 10th February 1988.

On 9th March 1988 three mice from each of groups A, B and C were bled and each of the remaining mice in groups A, B and C were challenged subcutaneously (s.c.) with 0.2 ml PBS containing $10^3$ plaque forming units of the Neudorfl isolate of TBEV.

The results were as follows:-

| Animal Group | Vaccine | No. of Survivors | % Survivors |
|---|---|---|---|
| A | 2 x vaccine + HGH | 9/9 | 100 |
| B | PBS + HGH | 0/9 | 0 |
| C | 1 x vaccine + HGH | 8/9 | 89 |
| A | 2 x vaccine | 9/9 | 100 |
| B | PBS | 0/9 | 0 |
| C | 1 x vaccine | 3/9 | 33.3 |

Example 2- Demonstration of Immunostimulant Effect

The effect of administering HGH on the effectiveness of a vaccine against Tick-Borne Encephalitis at sub-optimal doses was carried out using the protocol used in Example 1 except that the TBE vaccine was diluted 1:100 before use.

The results were as follows:-

| Animal Group | Vaccine | No. of Survivors | % Survivors |
|---|---|---|---|
| A | 2 x vaccine + HGH | 5/9 | 55 |
| B | PBS + HGH | 0/9 | 0 |
| C | 1 x vaccine + HGH | 2/9 | 22 |
| A | 2 x vaccine | 2/9 | 22 |
| B | PBS | 0/9 | 0 |
| C | 1 x vaccine | 2/9 | 22 |

Assuming that each mouse weighs, on average 20 g and an average 5 year old child weighs 40 kg; each mouse received the equivalent dose per kilogram of ten times the normal human dose of HGH when used to treat pituitary dwarfism. During childhood and on into adult life, the pituitary continuously excretes HGH, but at levels below that required to obtain the enhanced immunostimulant activity according to the invention. The invention therefore requires the administration of quantities of hormone sufficient to result in a plasma level of hormone above normal basal levels.

In the case of HGH, it is thus desirable to administer at least $10^{-2}$ units of HGH per kilogram of body weight, preferably from $10^{-2}$ to $10^{-1}$ units/kg.

Example 3 - Demonstration of Immunostimulant Effect

The experiment of Example 1 was repeated, except that the HGH used was a commercially available polypeptide produced by recombinant DNA technology.

The following results were obtained:

| Animal Group | Vaccine | No. of Survivors | % Survivors |
|---|---|---|---|
| A | 2 x vaccine + HGH | 9/9 | 100 |
| B | PBS + HGH | 0/9 | 0 |
| C | 1 x vaccine + HGH | 9/9 | 100 |

### Example 4 - Analysis of Mouse Sera For TBVE-Specific Antibodies

The experiment of Example 1 was repeated and blood samples were analysed for TBVE-specific antibodies.

The results obtained were as follows:

| Animal Group (f) | (g) ELISA titre at various times after vaccination | | |
|---|---|---|---|
| | 14 days(a) | 28 days(b) | 42 days(c) |
| A | 80 | 160 | 2560 |
| B | 80 | 80 | 320(d) |
| C | 160 | 80 | 1280 |
| A + HGH | 320 | 1280 | >10240 |
| B + HGH | 160 | 320 | 0(e) |
| C + HGH | 160 | 320 | >10240 |

### Notes

a. Blood sample taken when second shot of vaccine was given to group A.

b. Blood sample taken when virus challenge was given.

c. Blood sample taken at the end of the experiment.

d. Although all mice in this group are normally dead at this time, a sample from an occasional survivor was analysed.

e. No sample available as all mice died.

f. Groups are designated as in the tables of the survival data.

g. In this assay a titre of 300 or less would be considered a negative; therefore it appears that vaccine alone stimulates very little antibody production and even in the presence of HGH antibody titres are low. It is only after challenge with live virus that significant levels of antibody are detected. Although there is a stimulation in antibody production with HGH it is probably insufficient to account for the increased levels of protection that are observed.

### Example 5 - Dose Response Of The Ability Of HGH To Stimulate Protection

The dose response of the HGH preparation was tested by performing a series of protection experiments, identical in design to those described in Example 1, except that the HGH solution was diluted before addition to the vaccine preparation. A total of six experiments were performed and the data for two of them is presented below. Experiment A was performed with a 1:4 dilution of HGH, i.e. the highest dilution at which no reduction in the immunostimulant effect of HGH was observed. Experiment B was performed with a 1:40 dilution of HGH, i.e. the lowest dilution at which the immunostimulant effect of HGH was not observed.

### Experiment A

| Animal Group | Vaccine | No. of Survivors | % Survivors |
|---|---|---|---|
| A | 2 x vaccine + HGH | 9/9 | 100 |
| B | PBS + HGH | 0/9 | 0 |
| C | 1 x vaccine + HGH | 9/9 | 100 |

### Experiment B

| Animal Group | Vaccine | No. of Survivors | % Survivors |
|---|---|---|---|
| A | 2 x vaccine + HGH | 9/9 | 100 |
| B | PBS + HGH | 0/9 | 0 |
| C | 1 x vaccine + HGH | 4/9 | 44 |

By this series of experiments the "Stimulant dose$_{50}$" was estimated to be a 1:20 dilution of the original concentration of HGH, i.e. a concentration of 0.2 international units m/l.

From the results of Examples 1 and 2 it can be seen that HGH induces a marked immunostimulant action and enhances the immune response against TBE vaccine both using a less than optimal number of inoculations and using a less than optimal vaccine concentration.

Thus for TBE vaccine in the mouse protection model described above, HGH is capable of improving the protective efficacy of a single optimal dose of vaccine from 33% to 100% and is capable of improving the protective efficacy of two sub-optimal doses from 22% to 55%.

The results of Example 3 demonstrates that recombinant HGH is capable of exerting an immunostimulant effect. This establishes that HGH itself (as opposed to an incidental impurity in cadaver-derived material) is capable of exerting an immunostimulant effect.

The mechanism of this potentiation of the immune system is at present unknown, but experiments show only a slight increase in overall specific antibody levels. This would suggest that HGH might act directly on the stimulation of the cell mediated immune system.

Thus as shown in the results of Example 4, the administration of HGH resulted in no significant elevation of ELISA titre at 14 days, a marginal elevation of ELISA titre at 28 days and a marked elevation at 42 days after vaccination (i.e. 14 days after challenge with live virus).

It can be concluded that the effect of coadministering vaccine plus HGH does not result in an increase in antibody levels (compared to controls which receive only vaccine) and that consequently the cell mediated immune system is involved.

The method of the invention has considerable potential in human and animal therapy in enhancing the effectiveness of standard vaccines and enabling a significant degree of protection to be obtained using one-shot inocculation regimes.

## Claims

1. The use of a pituitary growth hormone in the manufacture of a pharmaceutical composition for eliciting an immunostimulant effect in a human or non-human animal.

2. The use as claimed in Claim 1 wherein the pituitary growth hormone is a human growth hormone.

3. The use as claimed in any preceding claim wherein the pituitary growth hormone is produced by recombinant DNA techniques and comprises a polypeptide which (i) has an amino acid sequence which is identical to a native pituitary growth hormone or (ii) has an amino acid sequence which differs from a native pituitary growth hormone by up to thirty percent of the native sequence, with the proviso that said poly-

7

EP 0 434 749 B1

peptide (ii) possesses at least one biological activity of native pituitary growth hormone.

4. The use according to any preceding claim wherein the immunostimulant effect is produced in a human or non-human animal in which it is desired to elicit or stimulate an immune response against a selected antigen, which antigen is capable of producing a protective effect.

5. The use according to Claim 4 wherein the selected antigen is a component of a vaccine.

6. The use according to Claim 4 or Claim 5 wherein the pituitary growth hormone is administered prior to, simultaneously with or subsequent to the administration of the antigen.

7. The use according to any of Claims 4 to 6 wherein the selected antigen is capable of producing a protective immunological effect against the causative organisms of herpes, rubella, Japanese encephalitis, Rift Valley Fever, yellow fever, measles, mumps, poliomyelitis, TBEV, louping I11, dengue fever, lassa fever, Central European Tick - borne encephalitis, Russian Spring-Summer encephalitis, Omsk Haemorrhagic Fever or Kyanasur Forest Disease, against the causative organism, of Wesselbron disease, or against HIV, influenza virus, para influenza virus, Epstein-Barr virus and parvovirus B19.

8. The use claimed in any preceding claim wherein the pituitary growth hormone is derived from (a) an animal species other than the one in which it is desired to produce an immunostimulant effect, or (b) from the same animal species as the one in which it is desired to produce an immunostimulant effect.

9. A pharmaceutical preparation for use in producing an immune response in a human or non-human animal comprising an antigen and at least one pituitary growth hormone.

10. A pharmaceutical preparation according to Claim 9 in the form of an injectable pharmaceutical preparation comprising an antigen, at least one pituitary growth hormone, and a pharmaceutically acceptable carrier or added ingredient for encouraging localised retention of the pituitary growth hormone at or adjacent the injection site.

11. A pharmaceutical preparation according to Claim 9 or Claim 10 wherein the pituitary growth hormone is as defined in Claim 2 or Claim 3.


**Patentansprüche**

1. Verwendung eines Hypophysen-Wachstumshormons zur Herstellung einer pharmazeutischen Zusammensetzung zur Auslösung eines immunstimulierenden Effektes in einem Menschen oder in einem Tier.

2. Verwendung nach Anspruch 1, in der das Hypophysen-Wachstumshormon ein menschliches Wachstumshormon ist.

3. Verwendung nach einem der vorhergehenden Ansprüche, in der das Hypophysen-Wachstumshormon durch rekombinante DNA-Techniken hergestellt wird und ein Polypeptid umfaßt, das (i) eine Aminosäuresequenz aufweist, die identisch mit einem nativen Hypophysen-Wachstumshormon ist oder (ii) eine Aminosäuresequenz aufweist, die von einem nativen Hypophysen-Wachstumshormon bis zu 30% von der nativen Sequenz verschieden ist, mit der Maßgabe, daß das Polypeptid (ii) mindestens eine biologische Aktivität des nativen Hypophysen-Wachstumshormons besitzt.

4. Verwendung nach einem der vorhergehenden Ansprüche, in der der immunstimulierende Effekt in einem Mensch oder in einem Tier erzeugt wird, in dem es erwünscht ist, eine Immunantwort gegen ein ausgewähltes Antigen auszulösen oder zu stimulieren, wobei das Antigen in der Lage ist eine protektive Wirkung zu erzeugen.

5. Verwendung.nach Anspruch 4, in der das ausgewählte Antigen ein Bestandteil eines Vaccins ist.

6. Verwendung nach Anspruch 4 oder 5, in der das Hypophysen-Wachstumshormon vor, gleichzeitig oder anschließend an die Verabreichung des Antigens verabreicht wird.

7. Verwendung nach einem der Ansprüche 4 bis 6, in der das Antigen in der Lage ist, einen protektiven immunologischen Effekt gegen die kausativen Organismen von Herpes, Rubella, japanische Encephalitis, Rift-Valley-Fieber, Gelbfieber, Masern, Mumps, Poliomyelitis, TBEV, Drehkrankheit, Dengue-Fieber, Lassafieber, mitteleuropäische zeckenverursachte Encephalitis, russische Frühjahrs-Sommerencephalitis, Omsker-Hämmorhagisches Fieber oder Kyasanurwald-Fieber, gegen die kausativen Organismen der Wesselbron-Krankheit oder gegen HIV, Influenzavirus, Parainfluenzavirus, Epstein-Barr-Virus und Parvovirus B19 zu erzeugen.

8. Verwendung nach einem der vorhergehenden Ansprüche, in der das Hypophysen-Wachstumshormon von (a) einer Tierspezies stammt, die verschieden ist, von der, in der die Erzeugung eines immunstimulierenden Effektes gewünscht ist, oder (b) von der gleichen Tierspezies stammt, wie die, in der die Erzeugung eines immunstimulierenden Effektes gewünscht ist.

9. Pharmazeutische Zubereitung zur Verwendung in der Erzeugung einer Immunantwort in einem Mensch oder Tier, die ein Antigen und mindestens ein Hypophysen-Wachstumshormon enthält.

10. Pharmazeutische Zubereitung nach Anspruch 9, in Form einer injizierbaren pharmazeutischen Zubereitung, die ein Antigen, mindestens ein Hypophysen-Wachstumshormon und einen pharmazeutisch annehmbaren Träger oder oder zugefügten Bestandteil zur Förderung einer begrenzten Retention des Hypophysen-Wachstumshormons auf der oder bei der Injektionsstelle enthält.

11. Pharmazeutische Zubereitung nach Anspruch 9 oder 10, in der das Hypophysen-Wachstumshormon das in Anspruch 1 oder 2 definierte ist.


**Revendications**

1. Utilisation d'une hormone de croissance hypophysaire dans la fabrication d'une composition pharmaceutique pour provoquer un effet immunostimulant chez un homme ou un animal non humain.

2. Utilisation suivant la revendication 1, dans laquelle l'hormone de croissance hypophysaire est une hormone de croissance humaine.

3. Utilisation suivant l'une quelconque des revendications précédentes, dans laquelle l'hormone de croissance hypophysaire est produite par des techniques d'ADN recombinant et comprend un polypeptide qui (i) a une séquence d'acides aminés qui est identique à celle d'une hormone de croissance hypophysaire naturelle, ou (ii) a une séquence d'acides aminés qui diffère jusqu'à 30% de la séquence naturelle d'une hormone de croissance hypophysaire naturelle, à condition que ce polypeptide (ii) possède au moins une activité biologique de l'hormone de croissance hypophysaire.

4. Utilisation suivant l'une quelconque des revendications précédentes, dans laquelle l'effet immunostimulant est produit chez un homme ou un animal non humain dans lequel on souhaite provoquer ou stimuler une réponse immune contre un antigène choisi, lequel antigène est capable de produire un effet protecteur.

5. Utilisation suivant la revendication 4, dans laquelle l'antigène choisi est un composant d'un vaccin.

6. Utilisation suivant les revendications 4 ou 5, dans laquelle l'hormone de croissance hypophysaire est administrée avant, pendant ou après l'administration de l'antigène.

7. Utilisation suivant l'une quelconque des revendications 4 à 6, dans laquelle l'antigène choisi est capable de produire un effet immunologique protecteur contre les organismes causatifs de l'herpes, la rubéole, l'encéphalite japonaise, la fièvre de la Vallée du Rift, la fièvre jaune, la rougeole, les oreillons, la poliomyélite, le TBEV, l'encéphalite écossaise, la dengue, la fièvre de Lassa, l'encéphalite transmise par les tiques d'Europe centrale, l'encéphalite russe verno-estivale, la fièvre hémorrhagique d'Omsk ou la maladie de la forêt de Kyanasur, contre l'organisme causatif de la malade de Wesselbron, ou contre les HIV, virus de la grippe, virus para-influenza, virus d'Epstein-Bar et parvovirus B19.

8. Utilisation suivant l'une quelconque des revendications précédentes, dans laquelle l'hormone de croissance hypophysaire provient de (a) une espèce animale autre que celle chez laquelle on désire produire un effet immunostimulant, ou (b) la même espèce animale que celle chez laquelle on désire produire un effet immunostimulant.

9. Préparation pharmaceutique utile pour produire une réponse immune chez un homme ou un animal non humain comprenant un antigène et au moins une hormone de croissance hypophysaire.

10. Préparation pharmaceutique suivant la revendication 9, sous la forme d'une préparation pharmaceutique injectable comprenant un antigène et au moins une hormone de croissance hypophysaire et un support acceptable du point de vue pharmaceutique ou un ingrédient ajouté pour favoriser la rétention localisée de l'hormone de croissance hypophysaire au site d'injection ou à son voisinage.

11. Préparation pharmaceutique suivant les revendications 9 et 10, dans laquelle l'hormone de croissance hypophysaire est telle que définie dans la revendication 2 ou la revendication 3,